(19) Europäisches Patentamt
European Patent Office
Office européen des brevets

(11) Publication number: **0 122 452**
**A1**

(12) # EUROPEAN PATENT APPLICATION

(21) Application number: 84102676.8

(22) Date of filing: 12.03.84

(51) Int. Cl.[3]: C 07 D 249/08
A 61 K 31/41

(30) Priority: 18.03.83 US 476681

(43) Date of publication of application:
24.10.84 Bulletin 84/43

(84) Designated Contracting States:
AT BE CH DE FR GB IT LI NL SE

(71) Applicant: SCHERING CORPORATION
2000 Galloping Hill Road
Kenilworth, New Jersey 07033(US)

(72) Inventor: Wright, John Jessen
691 Marigold Court
Evansville Indiana 47712(US)

(72) Inventor: Eliott, Arthur John
62 Seven Lakes Drive
Sloatsburg New York 10974(US)

(72) Inventor: Cooper, Alan Bruce
14 Adon Circle
West Caldwell New Jersey 07006(US)

(72) Inventor: Desai, Jagdish Armitlal
114 Styvesant Ave.
Jersey City New Jersey 07306(US)

(74) Representative: Antony, Fritz, Dr. et al,
c/o Scherico Ltd. P.O. Box 601 Töpferstrasse 5
CH-6002 Lucerne(CH)

(54) **Triazolyl- and imidazolyl-substituted fluoroalkane derivatives, process for their preparation and pharmaceutical compositions containing them.**

(57) The invention relates to novel compounds of the general formula

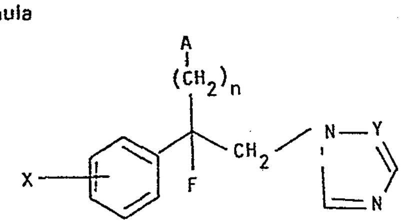

wherein n is zero or 1,

A is X-substituted phenyl or X-substituted thienyl (the phenyl and thienyl group containing 1 2 or 3 substituents X) or, provided n is 1 A can also be OR', SR', 1-imidazolyl or 1-(1, 2, 4-triazolyl);

R' is 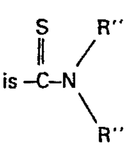 , 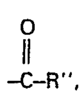,

lower alkyl, substituted or unsubstitued phenyl, or substituted or unsubstituted phenyl lower alkyl, wherein the substituents are 1 or 2 halogen atoms;

R'' and R''' are each lower alkyl, which can be the same or different;

X is halogen, lower alkoxy, or lower alkyl;

Y is N or CH; and

the pharmaceutically acceptable acid addition salts thereof;

to processes for the preparation thereof and to pharmaceutical compositions containing the compounds. The compounds are useful as antifungal agents.

EP 0 122 452 A1

# TRIAZOLYL- AND IMIDAZOLYL-SUBSTITUTED FLUOROALKANE DERIVATIVES, PROCESS FOR THEIR PREPARATION AND PHARMACEUTICAL COMPOSITIONS CONTAINING THEM

This invention relates to triazolyl- and imidazolyl-substituted fluoroalkane derivatives useful as anti-fungal agents and to processes for preparing them. The invention also relates to pharmaceuitcal and veterinary compositions comprising the triazolyl or imidazolyl compounds. Imidazolyl- and triazolyl- substituted propanols and ethanols are known from the following publications. U.K.-patent application 2,078,719, European patent applications 48 548, 36 153 and 54 974.

The invention relates to compounds of the general formula

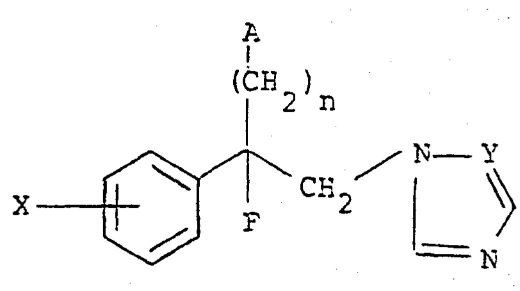

I

wherein n is zero or 1,

A is X-substituted phenyl or X-substituted thienyl (the phenyl and thienyl group containing 1, 2 or 3 substituents X) or, provided n is 1 A can also be OR', SR', 1-imidazolyl or 1-(1,2,4-triazolyl);

R' is 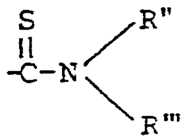 , 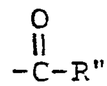, lower alkyl, substituted or unsubstitued phenyl, or substituted or unsubstituted phenyl lower alkyl, wherein the substituents are 1 or 2 halogen atoms;

R" and R"' are each lower alkyl, which can be the same or different;

X is halogen, lower alkoxy, or lower alkyl;

Y is N or CH; and

the pharmaceutically acceptable acid addition salts thereof.

As used herein, the term "lower alkyl" means straight or branched alkyl chains having 1 to 6 carbon atoms, e.g. methyl, ethyl, n-propyl, n-butyl, iso-butyl, pentyl and hexyl. Similarly, "lower alkoxy" means straight or branched alkoxy groups having 1 to 6 carbon atoms, e.g. methoxy, ethoxy, propoxy, iso-propoxy and butoxy. The term "halogen" means chlorine, bromine or fluorine. "Thienyl" means 2-thienyl or 3-thienyl.

"Pharmaceutically acceptable acid addition salts" means those salts formed with inorganic or organic acids, e.g. hydrochloric, nitric, sulfuric, acetic, p-toluenesulfonic, phosphoric, maleic or oxalic acid.

Preferred compounds of formula I are those wherein Y is nitrogen.

Of the preferred compounds of formula I wherein n is 1, more preferred are those wherein A is 1-(1,2,4)-triazolyl, especially 1,3-di-1-N-triazolyl compounds.

Of the preferred compounds of formula I wherein n is zero, more preferred are those wherein A is substituted phenyl.

Also preferred are compounds wherein X is halogen, particularly chlorine.

The present invention includes within its scope the method of eliciting an antifungal response in a human or animals, particularly warm-blooded animals, having fungal infections which comprises administering an antifungally effective amount of a compound of formula I or a pharmaceutical composition thereof. Parenteral (e.g. topical) administration is useful, however oral administration is preferred.

The compounds of formula I exhibit antifungal activity against such human and animal pathogens as Candida and Trichophyton, as demonstrated by conventional <u>in vivo</u> tests in animals, e.g. a mouse systemic infection model. These tests indicate the compounds of this invention to be orally active. In one such test, a chronic Candida kidney infection model in mice, infected mice treated with 100 mg/kg 2-(4-chlorophenyl)-2-fluoro-1,3-di-[1-(1,2,4-1<u>H</u>-triazolyl)]propane per day for ten days and sacrificed about 5 days after treatment ended showed a 15% lower number of colony forming units than mice treated similarly with ketoconazole, a commercially available oral anti-fungal agent.

Dosage level and mode of administration will vary in the judgement of the attending clinician according to the particular host and the type and severity of infection. In general, for oral administration the dosage range will be from about 100 to about 500 mg per day, in single or divided doses, with the preferred range being about 125 mg to about 250 mg per day active ingredient, combined with a suitable pharmaceutically acceptable carrier or diluent.

For the compounds of the invention tested, it is considered that they are effectively non-toxic at the dose-rates indicated.

Also included in this invention are pharmaceutical formulations comprising an antifungally effective amount of a compound of formula I in a pharmaceutically acceptable, non-toxic carrier, preferably for oral administration. Typical oral dosage forms include tablets, capsules, elixirs, suspensions and the like. These dosage forms include typical pharmaceutically acceptable carriers, e.g. sugars such as lactose or sucrose, starches such as corn starch cellulose and derivatives such as sodium carboxy cellulose or methyl cellulose, calcium phosphates, stearic acid, alkaline earth stearates such as magnesium stearate, and polyalkylene glycols.

It is also contemplated that the antifungal compounds of this invention may be administered to animals in need of such treatment via animal feeds or in drinking water conventially used for the animal being treated.

The compounds of formula I can be prepared according to processes known in the art.

A. Compounds of formula I, wherein n is 1, A is OR', SR', imidazolyl or 1,2,4-triazolyl;
R' is 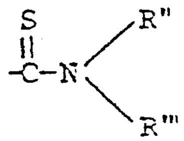 , 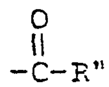, lower alkyl, substituted or unsubstitued phenyl, or substituted or unsubstituted phenyl lower alkyl, wherein the substituents are 1 or 2 halogen atoms;
R" and R"' are each lower alkyl, which can be the same or different;
X is halogen, lower alkoxy, or lower alkyl;
Y is N or CH;

can be prepared by reacting a compound of formula II

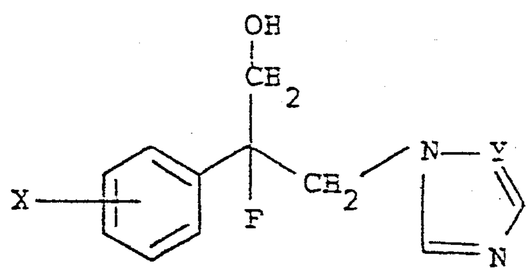

II

or a reactive derivative thereof with a compound A-H(III) or a reactive derivative thereof, wherein X, Y and A are as defined above in this paragraph.

A suitable reactive derivative of the compound of formula II is for example the corresponding trifluoromethylsulfonate, a suitable reactive derivative of the compound of formula III is for example the halide, preferably the chloride.

For the preparation of the compounds (I) defined above except for those wherein R' is 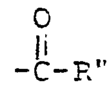, the process can be carried out in an inert solvent, such as for example dimethylformamide or tetrahydrofuran, in the presence of a base such as for example sodium hydride. For the preparation of compounds wherein A is OP', especially 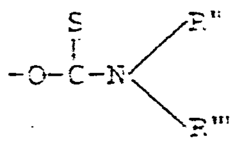 it is preferred to use a compound of formula II and a compound of formula III which is hal-R'.

Compounds, wherein R' is 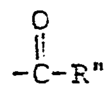 or lower alkyl can be prepared by usual acylation or alkylation reactions, respectively.

The starting compounds for process A are either known compounds or can be prepared according to known methods. The compounds of formula II can be prepared by reacting 1,2,4-triazole or imidazole with appropriately substituted α-bromomethylstyrene in an inert solvent (e.g. dimethylformamide) in the presence of a base such as sodium hydride, then heating with a peracid such as m-chloroperbenzoic acid in a solvent such as chloroform and then treating the resultant imidazolyl- or triazolyl-substituted styrene oxide with a reagent such as hydrogen fluoride-pyridine complex (Synthesis, 779, 780, 785 (1973) and ibd. 896 and 653 (1974); Chem. Commun., 451 (1976)) in a solvent such as chloroform to give the corresponding 2-fluoro-2-phenyl propanol derivative (II). If desired this compound can be transferred to its activated derivative, e.g. its trifluoromethylsulphonate.

B. Compounds of formula I wherein n is zero or 1, A is X-substituted phenyl or X-substituted thienyl or, provided n is 1 A can also be imidazolyl or 1,2,4-triazolyl;
X is halogen, lower alkoxy, or lower alkyl;
Y is N or CH;
can be prepared by fluorination of the corresponding tertiary alcohol of formula IV.

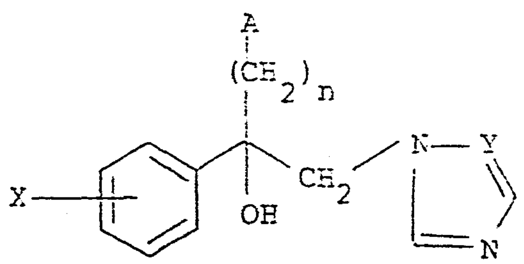

IV

This process can for example be performed with diethylaminosulfur trifluoride (DAST) in a solvent such as dichloromethane.

The starting compounds used in this process are either known compounds or can be prepared according to known processes.
1,3-Di-imidazolyl and 1,3-ditriazolyl-compounds of formula IV can for example be prepared by oxidation of X-substituted-α-bromomethylstyrene with a reagent such as m-chloroperbenzoic acid in a solvent such as dichloromethane to obtain the corresponding styrene oxide.
This styrene oxide is then coverted to the 1,3-di-1-N-triazolyl or 1,3-di-1-N-imidazolyl-2-hydroxy propane compound by reaction with 1,2,4-triazole or imidazole in an inert solvent such as dimethylformamide in the presence of a base such as sodium hydride.

Compounds of formula IV wherein A is X-substituted phenyl or X-substituted thienyl can be prepared for example by alkylation of X-substituted α-bromomethylstyrene with a reagent such as A-lithium in a solvent such as THF to obtain the corresponding X-substituted phenyl or X-substituted thienyl-styrene. This compound is then treated with a peracid such as m-chloroperbenzoic acid in a solvent such as chloroform to obtain the corresponding styrene oxide. This styrene oxide is then converted to the corresponding 1-phenyl or thienyl -3-(1-N-imidazolyl or 1-N-1,2,4-triazolyl) propane by reaction with 1,2,4-triazole or imidazole in an inert solvent such as DMF in the presence of a base such as sodium hydride.

Acid addition salts of the compounds of formula I are prepared according to known procedures, such as by neutralization of the free base with the appropriate acid.

Compounds of formula I wherein n is zero contain an asymetric center and accordingly exist in isomeric forms. The isomers can be isolated by methods known in the art.

Examples illustrating the preparation of compounds of formula I are given below.

## EXAMPLE 1

### 2-(4-CHLOROPHENYL)-2-FLUORO-3-[1-(1,2,4-1H-TRIAZOLYL)]-PROPYL DIMETHYLTHIOCARBAMATE

A. 2-(4-Chlorophenyl-1-[1-(1,2,4-H-triazolyl)]-2-propene

Add 6.9 g of 1,2,4-triazole to a stirred mixture of 4.0 g sodium hydride (60% in oil) and 100 ml of dimethylformamide. Cool this mixture to 5°C and add 23.1 g 1-bromo-2-(4-chlorophenyl)-2-propene dropwise so that the temperature does not exceed 10°C. Stir for 30 minutes and evaporate the solvent *in vacuo*. Partition the residue between 500 ml ethyl acetate and 300 ml water. Separate the organic layer, dry over magnesium sulfate and evaporate the solvent *in vacuo* to obtain 15.3 g of a colorless solid, mp 58-60°; $^1$H NMR ($CDCl_3$)δ 5.13 (S,3H), 5.57 (S,1H), 7.27 (S,4H), 7.89 (S,1H), 8.00 (S,1H).

B. 2-(4-Chlorophenyl)-2-fluoro-3-[1-(1,2,4-1H-triazolyl)]-1-propanol

Reflux a stirred solution of 11.6 g of the product of Step A and 12.0 g of *m*-chloroperbenzoic acid in 200 ml chloroform for 2 hours. Evaporate the solvent *in vacuo*, dissolve the residue in 75 ml of chloroform and pour the solution through a short silica gel column. Elute the product with a mixture of tetrahydrofuran (49 parts), hexane (49 parts) and triethylamine (2 parts). Evaporate the fractions containing the desired product *in vacuo* and dissolve the residue in 150 ml of chloroform. Transfer the solution to a polyethylene container and add 10 ml of hydrogen fluoride-pyridine complex. Stir the mixture at room temperature for 6 hours and dilute with 800 ml of chloroform. Wash with saturated sodium bicarbonate until all the acid is neutralized, dry the

solution over potassium carbonate and evaporate the solvent *in vacuo*. Triturate the residue with ether to obtain 6.8 g of product, mp 134-6°; 'H NMR (DMSO-D6) δ 3.80 (dd, J=6, 20 Hz, 2H), 4.87 (d, J-21 Hz, 2H), 5.43 (t, J-6Hz, OH), 7.40 (S, 4H), 7.85 (S, 1H), 8.30 (S, 1H).

C. 2-(4-Chlorophenyl)-2-fluoro-3-[1-(1,2,4-1*H*-triazolyl)]-propyl dimethylthiocarbamate

Add 2.55 g of the product of Step B to a stirred mixture of 0.4 g of sodium hydride (60% in oil) and 50 ml of dimethylformamide at 50°C. Stir mixture for 15 minutes, then add 1.23 g of dimethylthiocarbamoyl chloride. Stir for 1 hour more, then evaporate the solvent *in vacuo*. Partition the residue between 300 ml ethyl acetate and 200 ml water, separate the organic layer, dry over magnesium sulfate and evaporate the solvent *in vacuo* to obtain the title compound, m.p. 120-122°C.

## EXAMPLE 2

1-(2,4-DICHLOROBENZYLOXY)-2-(4-CHLOROPHENYL)-2-FLUORO-3-[1-(1,2,4-1*H*-TRIAZOLYL)]PROPANE HYDROCHLORIDE

Add 2.55 g of the product of Example 1, Step B to a stirred mixture of 0.4 g sodium hydride (60% in oil) and 50 ml DMF at 50°C. Stir for 15 minutes, then add 1.95 g 2,4-dichlorobenzyl chloride and stir at 50°C for 8 hours. Evaporate the solvent *in vacuo* and partition the residue between 300 ml ethyl acetate and 300 ml of water. Separate the organic layer, dry of magnesium sulfate and evaporate the solvent *in vacuo*. Dissolve the residue in 200 ml ether and add 10 ml saturated ethereal hydrogen chloride solution. Evaporate the solvent *in vacuo* to obtain the title compound, m.p. 168-178°.

EXAMPLE 3

2-(4-CHLOROPHENYL)-1-(4-CHLOROPHENYLTHIO)-2-FLUORO-3-[1-(1,2,4-1*H*-TRIAZOLYL)]PROPANE

A. 2-(4-Chlorophenyl)-2-fluoro-3-[1-(1,2,4-1*H* triazolyl)]-propyl trifluoromethanesulfonate

Add 0.40 ml of trifluoromethansulfonyl chloride to a stirred mixture of 0.75 g of the product of Example 1, Step B, 5 ml of methylene chloride and 0.6 ml of triethylamine at -40°C. Remove cold bath, stir for 20 minutes, and use the solution of the title compound directly in Step B.

B. 2-(4-Chlorophenyl)-1-(4-chlorophenylthio)-2-fluoro-3-[1-(1,2,4-1*H*-triazolyl)]propane hydrochloride

Add the solution of the product from Step A to a stirred mixture of 1.14 g 4-chlorothiophenol, 0.33 g sodium hydride (60% in oil) and 50 ml of tetrahydrofuran at 0°C. Stir for one hour at room temperature, evaporate the solvent *in vacuo* and partition the residue between 300 ml ethyl acetate and 200 ml water. Dry the organic solution over sodium sulfate and evaporate the solvent *in vacuo*. Dissolve the residue in 100 ml ether and add 5 ml saturated ethereal hydrogen chloride solution. Evaporate the solvent in vacuo to obtain the title compound, m.p. 166-170°C.

EXAMPLE 4

2-(4-CHLOROPHENYL)-1-(4-CHLOROBENZYLTHIO)-2-FLUORO-3-[1-(1,2,4-1*H*-TRIAZOLYL)]PROPANE HYDROCHLORIDE

Substitute an equivalent amount of 4-chlorobenzylmercaptan for 4-chlorothiophenol in Example 3, Step B to obtain the title compound, m.p. 163-166°C.

EXAMPLE 5

2-(4-CHLOROPHENYL)-1-(4-CHLOROPHENOXY)-2-FLUORO-3-[1-(1,2,4-1H-TRIAZOLYL)]PROPANE

Substitute an equivalent amount of 4-chlorophenol for 4-chlorothiophenol in Example 3, Step B to obtain the title compound.

EXAMPLE 6

2-(4-CHLOROPHENYL)-2-FLUORO-1-(1-IMIDAZOLYL)-3-[1-(1,2,4-1H-TRIAZOLYL]PROPANE

Substitute an equivalent amount of imidazole for 4-chlorothiophenol in Example 3, Step B to obtain the title compound, m.p. 171-173°C.

EXAMPLE 7

2-(4-CHLOROPHENYL)-2-FLUORO-1,3-DI-[1-(1,2,4-1H-TRIAZOLYL)]PROPANE

A. 4-Chloro-α-bromomethylstyrene

Reflux 94.4 g of 4-chloro-α-methylstyrene, 90.0 g of N-bromosuccinimide, and 50.0 ml of carbon tetrachloride at 160-170°C. (Cool as necessary to maintain gentle reflux conditions.) After 10-12 minutes, when all the N-bromosuccinimide is dissolved, cool the reaction mixture to just below reflux with an ice-bath, then allow to cool slowly over 3 hours. Separate the precipitated succinimide by filtration and evaporate the carbon tetrachloride and excess α-methylstyrene. Distill the mixture to obtain 84.8 g of a 70/30 mixture

of p-chloro-α-bromomethylstyrene and 4-chloro-α-methylß-bromostyrene respectively, b.p. 66-67°C (0.9 mm); $N^{20}D$ 1.5924; 'H NMR (90 MHz, $CDCl_3$) δ 7.36 (4H, superficial doublet, J=1.5Hz), 5.47 (2H, d, J=3Hz), 4.30 (2H,S).

B. <u>4-Chloro-bromomethylstyrene oxide</u>

Dissolve 8.7 g of 4-chloro-α-bromomethylstyrene in 50 ml of dichloromethane and cool in an ice bath. Add, dropwise and with stirring, a solution of 9.0 g of m-chloroperbenzoic acid dissolved in 100 ml of dichloromethane. Stir the resulting suspension overnight at room temperature. Decompose the excess reagent with 5% sodium sulfite, wash with 5% sodium bicarbonate solution followed by a water wash and dry the dichloromethane layer over magnesium sulfate. Evaporate the solvent at 50°C *in vacuo* to obtain 8.0 g of 4-chloro-α-bromomethylstyrene oxide: 'H NMR (90 MHz, $CDCl_3$) δ 7.42 (4H, S), 3.88 3.63 (ABq, 2H, J=10.8Hz), 3.21, 2.98 (ABq, 2H, J=5Hz).

C. <u>2-(4-Chlorophenyl)-2-hydroxy-1,3-di-1-[1-(1,2,4-1*H*-triazolyl)]propane</u>

Wash 10.8 g of sodium hydride (50% oil dispension) with hexane and add to 50 ml of dry N,N-dimethylformamide. To this suspension add a solution of 15.6 g 1,2,4-triazole in 75 ml of dry N,N-dimethylformamide at 5-10°C. Stir for 30 minutes at 25°C and add a solution of 14.0 g of 4-chloro-α-bromomethylstyrene oxide in 75.0 ml of N,N-dimethylformamide at 10°-15°C. Heat at 70°C for 2 days. Evaporate the solvent at 50°C *in vacuo*, add 100 ml of water to the residue and extract with 3 x 100 ml portions of dichloromethane. Dry the dichloromethane

layers over magnesium sulfate, filter and evaporate the solvent. Triturate the residue with ether and filter the solids to obtain 7.0 g of 2-(4-chlorophenyl)-2-hydroxy-1,3-di-1-[1-(1,2,4-1H-triazolyl)]propane: 'H NMR (90 MHz, $CDCL_3$/DMSO-D6) δ 8.10 (2H, S), 7.83 (2H, S), 7.30 (4H, superficial S), 5.99 (1H, S, exchanges with $D_2O$), 4.56 (4H, ABQ, J=15 Hz).

D. 2-(4-Chlorophenyl)-2-fluoro-1,3-di-[1-(1,2,4-1H-triazolyl)]propane

Dissolve 0.609 g of 2-(4-chlorophenyl)-2-hydroxy-1,3-di-[1-(1,2,4-1H-triazolyl)]propane in 20 ml of dichloromethane and cool to -10°C with a ice/methanol bath under dry conditions. Add diethylaminosulfur trifluoride dropwise while stirring. After the addition is over stir for 2 hours. Add saturated sodium bicarbonate solution slowly. Add 50 ml more dichloromethane and shake in a separatory funnel. Wash the dichloromethane layer twice with 50 ml portions of water. Dry over magnesium sulfate and evaporate off the dichloromethane to obtain 0.53 g of the title compound: 'H NMR (90 MHz, $CDCl_3$/DMSO-D6) δ 8.06 (2H, S), 7.87 (2H, S), 7.23 (4H, ABQ with further fine splitting, J=9Hz), 4.73 (4H, dd, $J_{hf}$=21 Hz, multiplet with f coupling); MS m/e (rel. intensity) 306.0 $M^+$, 4), 288.0 (12), 287.0 (6), 286.0 (36), 226.1 (12), 224.1 (45), 222.1 (22), 218.1 (17), 217.1 (19), 204.1 (11), 199.1 (10), 197.1 (32), 191.1 (13), 190.1 (9), 182.1 (5), 181.0 (31), 180.0 (12), 179.0 (100), 177.0 (11), 169.1 (10), 158.1 (13), 156.1 (40), 152.1 (14), 150.1 (16), 145.1 (10), 143.1 (32), 137.1 (11), 136.1 (11), 135.1 (13), 127.1 (16), 125.1 (44), 121.1 (15), 101.2 (20), 83.2 (12), 82.2 (24), 55.0 (24).

## EXAMPLE 8

1-(4-CHLOROPHENYL)-1-(2,4-DICHLOROPHENYL)-1-FLUORO-2-[1-(1,2,4-1H-TRIAZOLYL)]ETHANE

Dissolve 1.0 g of 1-(4-chlorophenyl)-1-(2,4-dichlorophenyl)-1-hydroxy-2-[1-(1,2,4-1H-triazolyl)]-ethane in 50 ml of dichloromethane at -10° and add 2 ml of diethylaminosulfur trifluoride with stirring. Stir for 2 hours, then add 100 ml of saturated sodium bicarbonate solution slowly. Separate the layers, dry the organic solution over magnesium sulfate and evaporate the solvent *in vacuo* to give the title compound.

In a manner similar to Examples 1 to 8, using appropriate reagents, the following compounds can be prepared:

2-(4-chlorophenyl)-1-ethoxy-2-fluoro-3-[1-(1,2,4-1H-triazolyl)]propane;

2-(4-chlorophenyl)-1-ethylthio-2-fluoro-3-[1-(1,2,4-1H-triazolyl)]propane;

2-(4-chlorophenyl)-1-(4-chlorophenylethylthio)-2-fluoro-3-[1-(1,2,4-1H-triazolyl)]propane;

2-(4-chlorophenyl)-1-(4-chlorophenylethoxy)-2-fluoro-3-[1-(1,2,4-1H-triazolyl)]propane;

2-(4-methoxyphenyl)-1-(4-chlorophenoxy)-2-fluoro-3-[1-(1,2,4-1H-triazolyl)]propane;

2-(4-ethylphenyl)-1-(4-chlorophenylthio)-2-fluoro-3-[1-(1,2,4-H-triazolyl)]propane;

2-(4-chlorophenyl-2-fluoro-1,3-di-(1-imidazolyl)propane;

2-(4-chlorophenyl)-1-(4-chlorophenyloxy)-2-fluoro-3-(1-imidazolyl)propane;

2-(4-chlorophenyl)-1-(4-chlorobenzyloxy)-2-fluoro-3-(1-imidazolyl)propane;

2-(4-chlorophenyl)-1-(4-chlorophenylthio)-2-fluoro-3-(1-imidazolyl)propane;

2-(4-chlorophenyl)-1-(4-chlorobenzylthio)-2-fluoro-3-(1-imidazolyl)propane;

2-(4-chlorophenyl)-1-propoxy-2-fluoro-3-(1-imidazolyl)-propane;

2-(4-chlorophenyl)-1-propylthio-2-fluoro-3-(1-imidazolyl)propane;

2-(4-methoxyphenyl)-1-(4-chlorophenyloxy)-2-fluoro-3-(1-imidazolyl)propane;

2-(4-ethylphenyl)-1-(4-chlorophenylthio)-2-fluoro-3-(1-imidazolyl)propane;

2-(4-chlorophenyl)-2-fluoro-3(1-imidazolyl)propyl dimethylthiocarbamate;

2-(4-chlorophenyl)-2-fluoro-3-[1-(1,2,4-1H-triazolyl)] propyl dimethyldithiocarbamate; and

2-(4-chlorophenyl)-2-fluoro-3-(1-imidazolyl)propyl dimethyldithiocarbamate.

2-(4-chlorophenyl)-1-(4-fluorophenyl)-2-fluoro-3-[1-(1,2,4-1H-triazolyl)]propane;

2-(4-chlorophenyl)-1-(4-methoxyphenyl)-2-fluoro-3-[1-(1,2,4-1H-triazolyl)]propane;

2-(4-chlorophenyl)-1-(4-ethylphenyl)-2-fluoro-3-[1-(1,2,4-1H-triazolyl)]propane;

2-(4-chlorophenyl)-1-(3-chloro-2-thienyl)-2-fluoro-3-[1-(1,2,4-1H-triazolyl)]propane;

2-(4-chlorophenyl)-1-(2-methyl-3-thienyl)-2-fluoro-3-[1-(1,2,4-1H-triazolyl)]propane;

2-(4-chlorophenyl)-1-(2-methoxy-3-thienyl)-2-fluoro-3-[1-(1,2,4-1H-triazolyl)]propane;

1-(4-chlorophenyl)-1-(4-fluorophenyl)-1-fluoro-2-(1-imidazolyl)ethane;

1-(4-chlorophenyl)-1-(4-methoxyphenyl)-1-fluoro-2-(1-imidazolyl)ethane;

1-(4-chlorophenyl)-1-(4-methylphenyl)-1-fluoro-2-(1-imidazolyl)ethane;

1-(4-chlorophenyl)-1-(3-chloro-2-thienyl)-1-fluoro-2-(1-imidazolyl)ethane;

1-(4-chlorophenyl)-1-(2-methyl-3-thienyl)-1-fluoro-2-(1-imidazolyl)ethane;

1-(4-chlorophenyl)-1-(2-methoxy-3-thienyl)-1-fluoro-2-(1-imidazolyl)ethane;

1-(4-chlorophenyl)-1-(4-fluorophenyl)-1-fluoro-2-[1-(1,2,4-1H-triazolyl)]ethane;

1-(4-chlorophenyl)-1-(4-methoxyphenyl)-1-fluoro-2-[1-(1,2,4-1H-triazolyl)]ethane;

1-(4-fluorophenyl)-1-(4-ethylphenyl)-1-fluoro-2-[1-(1,2,4-1H-triazolyl)]ethane;

1-(4-chlorophenyl)-1-(3-chloro-2-thienyl)-1-fluoro-2-[1-(1,2,4-1H-triazolyl)]ethane;

1-(4-chlorophenyl)-1-(3-methoxy-2-thienyl)-1-fluoro-2-[1-(1,2,4-1H-triazolyl)]ethane;

1-(4-chlorophenyl)-1-(2-methyl-3-thienyl)-1-fluoro-2-[1-(1,2,4-1H-triazolyl)]ethane;

1-(4-chlorophenyl)-1-(4-fluorophenyl)-1-fluoro-2-[1-(1,2,4-1H-triazolyl)]ethane;

1-(4-chlorophenyl)-1-(4-fluorophenyl)-1-fluoro-2-(1-imidazolyl)ethane;

1-(4-chlorophenyl)-1-(4-ethoxyphenyl)-1-fluoro-2-(1-imidazolyl)ethane;

1-(4-chlorophenyl)-1-(3-methylphenyl)-1-fluoro-2-(1-imidazolyl)ethane;

1-(4-chlorophenyl)-1-(3-chloro-2-thienyl)-1-fluoro-2-(1-imidazolyl)ethane;

1-(4-chlorophenyl)-1-(2-methyl-3-thienyl)-1-fluoro-2-(1-imidazolyl)ethane; and

1-(4-chlorophenyl)-1-(2-methoxy-3-thienyl)-1-fluoro-2-(1-imidazolyl)ethane.

The following are typical pharmaceutical formulations containing as the active ingredient (designated "Drug") the compounds of this invention. The active ingredient may preferably be 2-(4-chlorophenyl)-2-fluoro-1,3-di-[1-(1,2,4-1H-triazolyl)]propane or an equivalent amount of any of the other compounds of this invention especially 2-(4-chlorophenyl)-1-(4-chlorophenylthio)-2-fluoro-3-]1-(1,2,4-1H-triazolyl)]propane, or 2-(4-chlorophenyl)-2-fluoro-1-(1-imidazolyl)-3-[1-(1,2,4-1H-triazolyl)] propane.

FORMULATION 1

| Tablet | 125.00 mg. tab. |
|---|---|
| Drug | 125.00 mg. |
| Polyethylene glycol 6000 | 100.00 mg. |
| Sodium lauryl sulfate | 6.25 mg. |
| Corn starch | 30.00 mg. |
| Lactose, anhydrous | 87.25 mg. |
| Magnesium stearate | 1.50 mg. |

Procedure:

Heat the polyethylene glycol 6000 to 70-80°C. Mix the drug, sodium lauryl sulfate, corn starch, and lactose into the liquid and allow the mixture to cool. Pass the solidified mixture through a mill. Blend granules with magnesium stearate and compress into tablets.

FORMULATION 2

| Capsule | 250 mg capsule |
|---|---|
| Drug | 250.00 mg. |
| Lactose, anhydrous | 100.00 mg. |
| Corn starch | 50.00 mg. |
| Microcrystalline cellulose | 95.00 mg. |
| Magnesium stearate | 5.00 mg. |

Procedure:

Mix the first four ingredients in a suitable mixer for 10-15 minutes. Add the magnesium stearate and mix for 1-3 minutes. Fill the mixture into suitable two-piece hard gelatin capsules using an encapsulating machine.

Claims for designated countries other than Austria.

1. A compound of the general formula

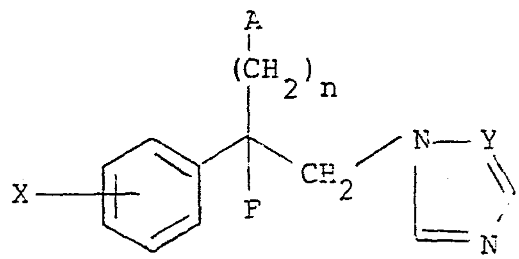

I

wherein n is zero or 1,

A is X-substituted phenyl or X-substituted thienyl (the phenyl and thienyl group containing 1, 2 or 3 X-substituents) or, provided n is 1 A can also be OR', SR', 1-imidazolyl or 1-(1,2,4-triazolyl);

R' is 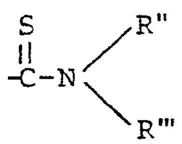 , 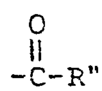, lower alkyl, substituted or unsubstitued phenyl, or substituted or unsubstituted phenyl lower alkyl, wherein the substituents are 1 or 2 halogen atoms;

R" and R"' are each lower alkyl, which can be the same or different;

X is halogen, lower alkoxy, or lower alkyl;

Y is N or CH; and

the pharmaceutically acceptable acid addition salts thereof.

2. A compound according to claim 1, wherein Y is nitrogen.

3. A compound according to claim 1 or 2 wherein X is halogen.

4. A compound according to claim 2 wherein n is 1 and A is 1-(1,2,4-triazolyl).

5. A compound according to any one of claims 1 to 3 wherein n is zero and A is substituted phenyl.

6. A compound according to claim 1 which is
2-(4-chlorophenyl)-2-fluoro-1,3-di-[1-(1,2,4-1H-triazolyl)]-propane,
2-(4-chlorophenyl)-2-fluoro-3-[1-(1,2,4-1H-triazolyl] propyldimethylthiocarbonate,
1-(2,4-dichlorobenzyloxy)-2-(4-chlorophenyl)-2-fluoro-3-[1-(1,2,4-1H-triazolyl)]propane,
2-(4-chlorophenyl)-1-(4-chlorophenylthio)-2-fluoro-3-[1-(1,2,4-1H-triazolyl)]propane,
2-(4-chlorophenyl)-1-(4-chlorobenzylthio)-2-fluoro-3-[1-(1,2,4-1H-triazolyl)]propane, or
2-(4-chlorophenyl)-2-fluoro-1-(1-imidazolyl)-3-[1-(1,2,4-1H-triazolyl)]propane.

7. Process for the preparation of compounds as defined in any one of claims 1 to 6 characterized in that
a) for the preparation of compounds of formula I, wherein n is 1, A is OR', SR', imidazolyl or 1,2,4-triazolyl;
R' is 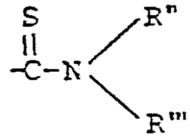 , 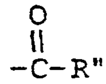, lower alkyl, substituted or unsubstitued phenyl, or substituted or unsubstituted phenyl lower alkyl, wherein the substituents are 1 or 2 halogen atoms;

0122452



R" and R"' are each lower alkyl, which can be the same or different;

X is halogen, lower alkoxy, or lower alkyl;

Y is N or CH;

a compound of formula II

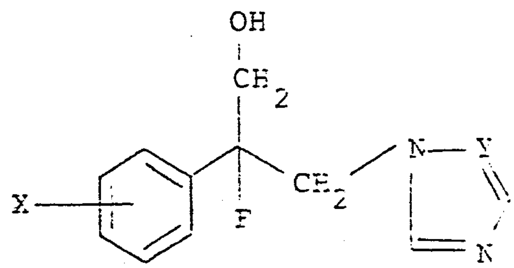

II

or a reactive derivative thereof is reacted with a compound A-H(III) or a reactive derivative thereof, wherein X, Y and A are as defined above;

b) for the preparation of compounds of formula I wherein n is zero or 1, A is X-substituted phenyl or X-substituted thienyl or, provided n is 1 A can also be imidazolyl or 1,2,4-triazolyl;

X is halogen, lower alkoxy, or lower alkyl;

Y is N or CH;

a corresponding tertiary alcohol of formula IV

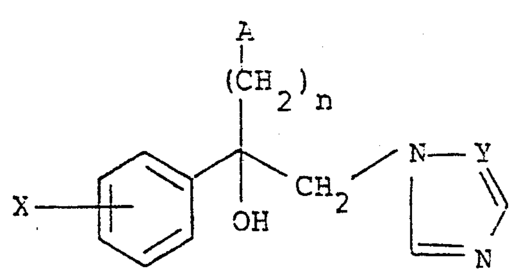

IV

is fluorinated;

followed if desired by forming a pharmaceutically acceptable acid addition salt.

8. A pharmaceutical composition useful for inhibiting the growth of fungi which comprises an antifungally effective amount of a compound of any one of claims 1 to 6, preferably in admixture with a pharmaceutically acceptable carrier.

9. A composition according to claim 8 which is suitable for oral use.

Claims for Austria

1. Process for the preparation of a compound of the general formula

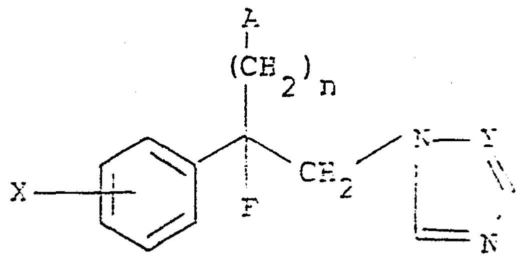

I

wherein n is zero or 1,
A is X-substituted phenyl or X-substituted thienyl (the phenyl and thienyl group containing 1, 2 or 3 X-substituents) or, provided n is 1 A can also be OR', SR', 1-imidazolyl or 1-(1,2,4-triazolyl);
R' is 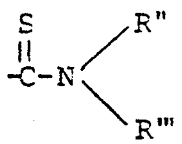 , 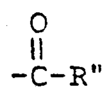, lower alkyl, substituted or unsubstitued phenyl, or substituted or unsubstituted phenyl lower alkyl, wherein the substituents are 1 or 2 halogen atoms;
R" and R''' are each lower alkyl, which can be the same or different;
X is halogen, lower alkoxy, or lower alkyl;
Y is N or CH; and
the pharmaceutically acceptable acid addition salts thereof, characterized in that

a) for the preparation of compounds of formula I, wherein n is 1, A is OR', SR', imidazolyl or 1,2,4-triazolyl;

R' is 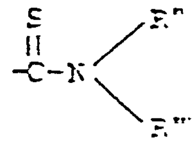 , 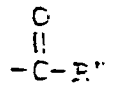, lower alkyl, substituted or unsubstitued phenyl, or substituted or unsubstituted phenyl lower alkyl, wherein the substituents are 1 or 2 halogen atoms,

R" and R‴ are each lower alkyl, which can be the same or different;

X is halogen, lower alkoxy, or lower alkyl;

Y is N or CH;

a compound of formula II

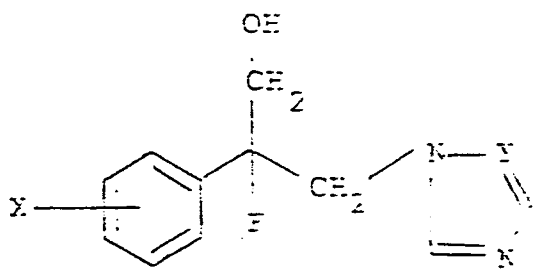

II

or a reactive derivative thereof is reacted with a compound A-H(III) or a reactive derivative thereof, wherein X, Y and A are as defined above;

b) for the preparation of compounds of formula I wherein n is zero or 1, A is X-substituted phenyl or X-substituted thienyl or, provided n is 1 A can also be imidazolyl or 1,2,4-triazolyl;

X is halogen, lower alkoxy, or lower alkyl;

Y is N or CH;

a corresponding tertiary alcohol of formula IV

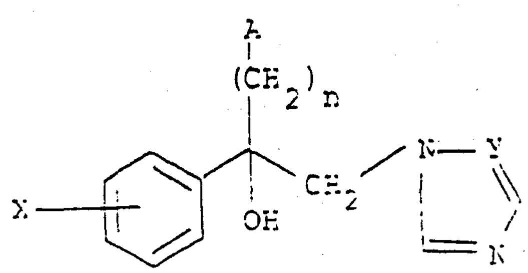

IV

is fluorinated,
followed if desired by forming a pharmaceutically acceptable acid addition salt.

2. A process according to claim 1, characterized in that a compound (I) is prepared wherein Y is nitrogen.

3. A process according to claim 1 or 2 characterized in that a compound (I) is prepared wherein X is halogen.

4. A process according to claim 2 characterized in that a compound (I) is prepared wherein n is 1 and A is 1-(1,2,4-triazolyl).

5. A process according to any one of claims 1 to 3 characterized in that a compound (I) is prepared wherein n is zero and A is substituted phenyl.

6. A process according to claim 1 characterized in that a compound (I) is prepared which is 2-(4-chlorophenyl)-2-fluoro-1,3-di-[1-(1,2,4-1H-triazolyl)]-propane,
2-(4-chlorophenyl)-2-fluoro-3-[1-(1,2,4-1H-triazolyl]propyldimethylthiocarbonate,
1-(2,4-dichlorobenzyloxy)-2-(4-chlorophenyl)-2-fluoro-3-[1-(1,2,4-1H-triazolyl)]propane,
2-(4-chlorophenyl)-1-(4-chlorophenylthio)-2-fluoro-3-

[1-(1,2,4-1H-triazolyl)]propane, 2-(4-chlorophenyl)-1-(4-chlorobenzylthio)-2-fluoro-3-[1-(1,2,4-1H-triazolyl)]propane, or 2-(4-chlorophenyl)-2-fluoro-1-(1-imidazolyl)-3-[1-(1,2,4-1H-triazolyl)] propane.

7. A process for the preparation of a pharmaceutical composition useful for inhibiting the growth of fungi which comprises an antifungally effective amount of a compound of any one of claims 1 to 6, preferably in admixture with a pharmaceutically acceptable carrier.

8. A process according to claim 7 characterized in that a composition is prepared which is suitable for oral use.

European Patent Office

# EUROPEAN SEARCH REPORT

0122452
Application number

EP 84 10 2676

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| D,A | EP-A-0 036 153 (BASF)<br>* Claims 1, 9 *<br>--- | 1,8 | C 07 D 249/08<br>A 61 K 31/41 |
| A | EP-A-0 044 605 (ICI)<br>* Claims 1, 9 * & GB-A-2 078 719 (Cat. D, A)<br>--- | 1,8 | |
| D,A | EP-A-0 054 974 (SUMITOMO CHEMICAL)<br>* Claim 1 *<br>--- | 1 | |
| A | DE-A-2 645 496 (BASF)<br>* Claim 1 *<br>----- | 1 | |
| | | | TECHNICAL FIELDS SEARCHED (Int Cl. ³) |
| | | | A 61 K 31/41<br>C 07 D 249/08<br>C 07 D 403/06 |

The present search report has been drawn up for all claims

| Place of search | Date of completion of the search | Examiner |
|---|---|---|
| BERLIN | 29-05-1984 | KNAACK M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82